# FASCICULE DE BREVET EUROPEEN

(11) **EP 4 539 852 B1**
(45) Date de publication et mention de la délivrance du brevet: **24.12.2025**
(21) Numéro de dépôt: 23731706.0
(22) Date de dépôt: 14.06.2023
(51) Int. Cl.: A61K 31/575, A61P 25/06, A61P 25/08, A61P 25/30, A61P 25/32

(54) **COMPOSES DERIVES DE STEROLS POUR LE TRAITEMENT D'UNE PATHOLOGIE LIEE A UN DEFICIT MITOCHONDRIAL**
STEROLDERIVATE ZUR BEHANDLUNG VON MIT MITOCHONDRIALEM DEFIZIT VERBUNDENEN KRANKHEITEN
STEROL DERIVATIVES FOR TREATING DISORDERS LINKED TO A MITOCHONDRIAL DEFICIT

(30) Priorité: 14.06.2022 BE 202205469
(43) Date de publication de la demande: 23.04.2025
(73) Titulaire: Dendrogenix, 4000 Liège (BE)
(72) Inventeur: SILVENTE, Stéphane, 4000 LIEGE (BE); CARON, Nicolas, 4560 Clavier (BE); RIVES, Arnaud, 4130 Tilff (BE); MARLIER, Quentin, 5080 Emines (BE); MICHAUX, Hélène, 4000 LIEGE (BE); MOSCA, Dario, 5020 Namur (BE); GILSOUL, Maxime, 4102 Ougrée (BE)
(74) Mandataire: Loyer & Abello
(86) Numéro de dépôt international: PCT/EP2023/065981
(87) Numéro de publication internationale: WO 2023/242281

(56) Documents cités:
- WO-A2-2016/016518
- CN-A- 114 206 347
- FR-A1- 2 838 741
- US-A1- 2010 098 678
- SHADEN A.M. KHALIFA ET AL: "The novel steroidal alkaloids dendrogenin A and B promote proliferation of adult neural stem cells", BIOCHEMICAL AND BIOPHYSICAL RESEARCH COMMUNICATIONS, vol. 446, no. 3, 1 April 2014 (2014-04-01), Amsterdam NL, pages 681 - 686, XP055687522, ISSN: 0006-291X, DOI: 10.1016/j.bbrc.2013.12.134
- DE MEDINA PHILIPPE ET AL: "Oxysterols are potential physiological regulators of ageing", AGEING RESEARCH REVIEWS, ELSEVIER, AMSTERDAM, NL, vol. 77, 26 March 2022 (2022-03-26), XP087019365, ISSN: 1568-1637, DOI: 10.1016/J.ARR.2022.101615
- DIAS IRUNDIKA HK ET AL: "Localisation of oxysterols at the sub-cellular level and in biological fluids", JOURNAL OF STEROID BIOCHEMISTRY & MOLECULAR BIOLOGY, ELSEVIER SCIENCE LTD., OXFORD, GB, vol. 193, 10 July 2019 (2019-07-10), XP085804843, ISSN: 0960-0760, DOI: 10.1016/J.JSBMB.2019.105426
- BARNABÉ-HEIDER F ET AL: "Stem cells for spinal cord repair", CELL STEM CELL, ELSEVIER, CELL PRESS, AMSTERDAM, NL, vol. 3, no. 1, 3 July 2008 (2008-07-03), pages 16 - 24, XP009542125, ISSN: 1934-5909, DOI: 10.1016/J.STEM.2008.06.011

## Description

### Domaine technique

L'invention se rapporte au domaine des pathologies liées à un déficit mitochondrial. Plus précisément, l'invention concerne des composés dérivés de stérol de formule (I) et une composition pharmaceutique comprenant des composés dérivés de stérols de formule (I) ou un sel pharmaceutiquement acceptable d'un tel composé, pour leur utilisation dans la prévention, l'amélioration et/ou le traitement d'une pathologie liée à un déficit mitochondrial choisie parmi le groupe constitué par l'autisme, les lésions de la moelle épinière, la sclérose en plaque, l'épilepsie, la migraine, les pathologies mentales liées à la consommation d'alcool, l'ataxie, les neuropathies, les troubles cérébraux et pulmonaires liés à la consommation du tabac, le syndrome de MERRF et le syndrome de NARP.

### Arrière-plan technologique

La mitochondrie est un organite fondamental présente dans la plupart des cellules eucaryotes. Leur rôle principal est de fournir aux cellules l'énergie nécessaire pour assurer leur survie et les fonctions qu'elles sont censées accomplir, en transformant l'oxygène et la nourriture en source d'énergie tel que l'adénosine triphosphate (ATP). Étant donné ce rôle fondamental joué par la mitochondrie pour la cellule, une multitude de pathologies sont associées à une dysfonction mitochondriale, affectant principalement les systèmes qui nécessitent de grandes quantités d'énergie, tels que les muscles, le cerveau ou le foie.

Une mitochondrie défectueuse ou endommagée produira de l'énergie de façon défectueuse également. Il existe des maladies mitochondriales dites primaires (héritées) ou secondaire (acquises). Dans les deux cas, les mitochondries ne fonctionneront plus correctement et entraineront différentes pathologies. Ces pathologies ont un lien avec l'activité de la mitochondrie, la dynamique de la mitochondrie (biogenèse, fusion, fission) et le transport axonal. Les maladies mitochondriales ou mitochondropathies regroupent un ensemble disparate de maladies en rapport avec un trouble de la chaine respiratoire mitochondriale.

Il existe une multitude de pathologies dans lesquelles sont observés des défauts d'activité mitochondriale et/ou de dynamique mitochondriale et/ou de transport axonal, comme notamment l'autisme, les lésions de la moelle épinière, la sclérose en plaque, l'épilepsie ou encore la migraine (Cleveland Clinic, *Mitochondrial diseases, https :*//*my.clevelandclinic.org*/*health*/*diseases*/*15612-mitochondrial-diseases).*

Mis à part des moyens naturels tels que faire de l'exercice ou adopter un mode de vie sain (éviter l'alcool, la privation de sommeil, faire des régimes alimentaires drastiques,...), il n'existe à l'heure actuelle que très peu de molécules capables d'améliorer le fonctionnement des mitochondries de manière générale. Le coenzyme Q10, la vitamine B (la thiamne, la riboflavin) l'acide Alpha lipoic, la L-carnitine, la créatine, la L-arginine, L'idebenone, le KH176, l'Elamipretide, le Bezafibrate, le re-sveratrol, l'omaveloxolone, la rapamycin (brevet US107...), des précurseurs du Nico-tinamide Adénine Dinucléotide (NAD+) sont régulièrement citées comme potentiels traitement aux maladies mitochondriales mais aucun d'eux n'a franchi toutes les phases cliniques. Seule la vitamine B12 peut être administrée pour le moment (Treatment for mitochodrial diseases, Tongling Liufu et al., September 9, 2020, https ://doi.org/10.1515/revneuro-2020-0034*;* Molecular Genetics and Metabolism, vol. 131, september-october 2020, pages 1-13, Clinical trials in mitochondrial disorders, an update, Mohammed Almannai et al., doi : 10.1016/j.ymgme.2020.10.002*).* Aussi les composés de type zolpidem ou butyrate ont été divulgués (US10,792,287 et document US10,272,056).

Il n'existe donc actuellement aucune thérapie approuvée par la FDA qui cible le traitement du dysfonctionnement mitochondrial (Trends in Molecular Medicine, Special issue : Mitochondria - from diagnosis to treatment review, vol.26, issue 1, pages 40-57, January 01, 2020, https ://doi.org/10.1016/j.molmed.2019.09.002).

Les mitochondropathies sont la conséquence majoritairement de mutations ou délétion dans des gènes codant des protéines mitochondriales. La fonction mitochondriale peut donc se trouver déficitaire soit par mutation génétique soit par atteinte mitochondriale acquise et iatrogène. Améliorer ou restaurer la fonction mitochondriale lorsque celle-ci est déficitaire est donc un défi fondamental dans la lutte contre toutes ces pathologies ou du moins contre quelques pathologies identifiées comme étant la conséquence d'un déficit mitochondrial.

Le spectre des troubles autistiques est une maladie neuro-développementale menant à des défauts de comportements sociaux ainsi qu'à des troubles cognitifs. Des dysfonctionnements mitochondriaux sont à présents considérés comme cause possible de ce trouble, notamment des défauts de dynamique mitochondriale (fission mitochondriale), de biogenèse mitochondriale ou encore de transport axonal de ces mitochondries (Seminar in Pediatric Neurology, vol. 25, October 2020, Richard E. Frye et al., Mitochondrial dysfunction in autism spectrum disorder : unique abnormalities and targeted treatments, https ://doi.org/10.1016/j.spen.2020.100829).

Lors de lésions de la moelle épinière, les axones dégénèrent. De plus en plus de preuves montrent que les mitochondries jouent un rôle fondamental dans les processus de dégénération/régénération de ces axones, que cela soit au niveau de l'activité mitochondriale, de leur dynamique ou du transport axonal des mitochondries (Frontiers in aging neuroscience, 8 Marsh 2021, Biyao Wang et al., Mitochondrial behavior in axon degeneration and regeneration, https ://doi.org/10.3389/fnagi.2021.650038).

Le sclérose en plaque est une maladie dans laquelle une démyélinisation des nerfs conduit à des dommages axonaux et à divers symptomes neurologiques. Dans cette pathologie, il a également été observé des défauts d'activité et de transport mitochondrial (Immunology and Inflammation, Neuroscience, February 10, 2021, Sina C Rosenkranz et al., Enhancing mitochondrial activity in neurons protects against neurodegeneration in a mouse model of multiple sclerosis, https ://elifesciences.org/articles/61798).

Une dysfonction mitochondriale a aussi été identifiée comme une cause potentielle de l'épilepsie (European Journal of Pediatric Neurology, vol.24, pages 47-52, January 01, 2020, Albert Lim et al., The mitochondrial epilepsies, https ://www.ejpn-journal.com/article/S1090-3798(19)30441-6/fulltext). Il a été également démontré que des antioxydants ciblant le stress oxydatif mitochondrial pouvaient jouer un rôle de neuroprotecteurs prometteurs pour l'épilepsie (Oxydative Medecine and Cellular Longevity, vol. 2020, Article ID 6687185, Nan Yang et al., Antioxidants targeting mitochondrial oxidative stress : promising neuro-protectants for epilpesy, https ://doi.org/10.1155/2020/6687185). Des crises répétées de migraine sont régulièrement associées à des modifications métaboliques de régions cérébrales spécifiques. Ces modifications métaboliques résultent de défauts de la fonction mitochondriale présents dans ces régions (Headache : The journal of head and face pain, 2018, vol.58 : pages 45-52, Kraya T. et al., Prevalance of headache in patients with mitochondrial disease : a cross-sectional study, https ://americanheadachesociety.org/news/journal-headache-mitochondrial-disease/*).*

Une dysfonction mitochondriale a été décrite dans les pathologies mentales liées à la consommation d'alcool. Plusieurs pathologies mentales peuvent être associées à la consommation d'alcool telles qu'une dépression majeure indépendante, un désordre bipolaire, de l'anxiété ou des troubles de la personnalité (Shivani, R., Goldsmith, R. J., & Anthenelli, R. M. (2002). Alcoholism and Psychiatric Disorders: Diagnostic Challenges. Alcohol Research & Health, 26(2), 90-98). Il a été décrit un mécanisme dû à la toxicité de l'éthanol sur les neurones du cerveau dans lequel la mitochondrie est le médiateur principal et dans lequel les altérations mitochondriales sont corrélées avec la sévérité de la consommation d'éthanol. Ainsi, l'amélioration de la santé des mitochondries des cellules cérébrales est considérée comme une cible thérapeutique potentielle pour traiter les pathologies associées à l'éthanol (Ethanol Consumption Affects Neuronal Function : Role of the Mitochondria, Cheril Tapia-Rojas et al., Decembrer 20, 2017, DOI : 10.5772/intechopen.71611). Il a également été démontré que la consommation d'alcool chez les adolescents a des répercussions importantes sur la bioénergie mitochondriale pendant l'âge adulte, et que ce n'est pas un changement transitoire jusqu'à l'arrêt de la consommation comme pensé auparavant (Neuroscience, vol 406, 15 May 2019, pages 356-368, Cheril Tapia-Rojas et al., Adolescence binge alcohol consumption induces hippocampal mitochondrial impairment that persists during the adulthood).

L'ataxie est une maladie neuromusculaire qui consiste en un manque de coordination fine des mouvements volontaires. Elle est liée à une atteinte du système nerveux. Parmi les ataxies on retrouve en lien avec un déficit mitochondriale le syndrome de Friedreich (Molecular and Cellular Neuroscience, vol. 102, January (2020), Anna Stepanova et al., Mitochondrial dysfunction in neurons in Freidrich's ataxia, https :/lwww.sciencedirect.com/science/article/abs/pii/S1044743119301964?via%3Dihub*), les télangiectasies (*Scientific Report 9, 4782 (2019), Blignaut, M. et al., Ataxia-Telangiectasia Mutated is located in cardiac mitochondria and impacts oxidative phosphorylation, https ://www.nature.com/articles/s41598-019-41108-1); l'ataxie spi-nocérébelleuse (Cell Reports, vol. 26, issue 5, pages 1189-1202, January 29, 2019, Metabolic and organelle morphology defects in mice and human patiients define spi-nocereballar ataxia type 7 as a mictochondrial disease, https ://www.cell.com/cell-reports/fulltext/S22111247(19)300373?_returnURL=https% 3A%2F%2Flinkinghub.elsevier.com%2Fretrieve%2Fpii%2FS22111247193 00373%3 Fshowall%3Dtrue#relatedArticles*).*

Les neuropathies et myopathies sont également démontrées avoir un lien avec un dysfonctionnement mitochondrial (Mitochondrion, 56 (2021), 52-61, Jian-Qiang Lu et al., Mitochondrial neuropathy and neurogenic features in mitochondrial myopat,. https://doi.org/10.1016/j.mito.2020.11.005*).*

Les troubles cérébraux et pulmonaires liés à la consommation du tabac ont été démontrés dans de nombreuses publications avec un impact de la nicotine ou de la fumée du tabac sur les différentes activités mitochondriales. La mitochondrie est présentée comme une cible possible de l'action de la nicotine dans le cerveau (Journal of Bioenergetics and Biomembranes, 51, 259-276 (2019), Dominika Malinska et al., Mitochondria as a possible target for nicotine action*)* et des perturbations dans la fonction et la structure mitochondriale des cellules épithéliales des poumons ont été démontrées par l'action de la fumée de cigarette (American Journal of Physiology, vol. 318, n°1, 7 January 2020, Mathyar Aghapour et al., Mitochondria : at the crossroads of regulating lung epithelial cell function in chronic obstructive pulmonary disease, https://doi.org/10.1152/ajplung.00329.2019).

Enfin, le Syndrome de MERRF (Myoclonic Epilepsy with Ragged Red Fibers) et le syndrome de NARP (Neuropathie, Ataxie et Rétinite Pigmentaire) sont tous deux en relation avec un déficit mitochondrial (Elsevier, Biochimica et Biphysica Acta (BBA) Molecular basis of Disease, vol. 1866, issue 6, June 1, 2020, Marina Vilanueva-Paz et al., Parkin-mediated mitophagy and autophagy flux disruption in cellular models of MERRF syndrome *;* Elsevier, The international Journal of Biochemistry & Cell Biology, vol. 45, issue A, January 2013, pages 141-150, Magdanela Lebiedzinska et al., Dirupted ATP synthase activity and mitochondrial hyperpolarisation-dependent oxidative stress is associated with p66Shc phosphorylation in fibroblasts of NAR patients*).*

Le document US 10,792,287 décrit l'utilisation de composés pharmaceutiques (zolpidene avec en complément rapamycine ou idebedone) pour leur effet bénéfique sur la production mitochondriale d'ATP, l'inhibition de l'inflammation et pour traiter différentes pathologies mitochondriales telles que la perte de la vision et la neuropathie optique héréditaire de Leber.

Le document US 10,272,056 décrit aussi l'utilisation de composés de type butyrate pour leurs effets bénéfiques sur la biogenèse mitochondriale, l'augmentation de la masse mitochondriale, l'augmentation de la production d'ATP et de leur efficacité dans plusieurs pathologies telles que les maladies neurodégénératives, cardiovas-culaires, neuro-métaboliques, musculaires, rénales, métaboliques et certains syndromes particuliers tels que le syndrome de MELAS (Mitochondrial Encephalopathy with Lactic Acidosis and Stroke-like episodes) et le syndrome de MERRF.

La demanderesse s'est aperçue de manière surprenante qu'un composé dérivé de stérols de formule (I) ou un sel pharmaceutiquement acceptable d'un tel composé, est utile dans la prévention, l'amélioration et/ou le traitement d'une pathologie liée à un déficit mitochondrial due soit à un déficit de la dynamique et/ou du transport et/ou de l'activité mitochondriale. Plus précisément, la demanderesse s'est aperçue qu'un composé dérivé de stérols de formule (I) ou un sel pharmaceutiquement acceptable d'un tel composé, est utile dans la prévention, l'amélioration et/ou le traitement d'une pathologie liée à un déficit mitochondrial choisie parmi le groupe constitué par l'autisme, les lésions de la moelle épinière, la sclérose en plaque, l'épilepsie, la migraine, les pathologies mentales liées à la consommation d'alcool, l'ataxie, les neuropathies, les troubles cérébraux et pulmonaires liés à la consommation du tabac, le syndrome de MERRF et le syndrome de NARP.

### Résumé

L'invention a pour objet de fournir des composés dérivés de stérol et une composition pharmaceutique les comprenant pour leur utilisation pour la prévention, l'amélioration et/ou le traitement d'une maladie liée à un déficit mitochondrial choisie parmi le groupe constitué par l'autisme, les lésions de la moelle épinière, la sclérose en plaque, l'épilepsie, la migraine, les pathologies mentales liées à la consommation d'alcool, l'ataxie, les neuropathies, les troubles cérébraux et pulmonaires liés à la consommation du tabac, le syndrome de MERRF et le syndrome de NARP.

Pour cela, l'invention fournit un composé de formule (I) : 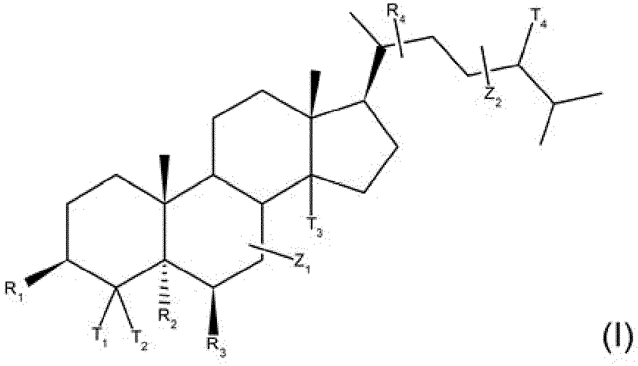 dans laquelle
R₁ = OH, F, OCₙH₂ₙ₊₁, OC(O)R, OC(O)OR, OC(O)NHR ou OP(O)(OR)₂ avec R = H ou CₙH₂ₙ₊₁, avec 1≤ n ≤ 8 ;
R₂ = H ou OH;
R₃ = -NR₅R₆,
R₅ étant H ou -(CH₂)₃NH₂, et
R₆ étant pris dans le groupe formé par -(CH₂)₃NR₇(CH₂)₄NHR₇ , -(CH₂)₃NHR₇ , -(CH₂)₄NHR₇, -(CH₂)₄N R₇(CH₂)₃NHR₇, -(CH₂)₃N R₇(CH₂)₄N R₇(CH₂)₃NHR₇, -(CH₂)₂ -imidazol-4-yl; -(CH₂)₂-indol-3-yl; avec R₇ = H, C(O)OCH₃ ou C(O)OC(CH₃)₃; et
R₄ = H ou OH en position 20, 22, 24, 25, 26 ou 27, positionné de façon à créer un centre asymétrique de configuration R ou S ;
Z₁ et Z₂ représentent chacun le nombre de doubles liaisons entre les atomes de carbone C7 et C8 et C22 et C23 respectivement (soit 0 soit 1) ; T₁, T₂ et T₃ = H ou CH₃ indépendamment les uns des autres ; T₄ = H, CH₃, C₂H₅ positionné de façon à obtenir un centre asymétrique de configuration R ou S en position 24 ;
pour son utilisation dans le traitement d'une pathologie liée à un déficit mitochondrial choisie parmi le groupe constitué par l'autisme, les lésions de la moelle épinière, la sclérose en plaque, l'épilepsie, la migraine, les pathologies mentales liées à la consommation d'alcool, l'ataxie, les neuropathies, les troubles cérébraux et pulmonaires liés à la consommation du tabac, le syndrome de MERRF et le syndrome de NARP.

La mention (O) dans la définition d'un radical selon la formule (I) signifie que l'oxygène est lié par deux liaisons.

Le composé de formule (I) et défini par : Z₁ = Z₂ = 0 ; R₁ = R₂ = OH ; R₄ = H ; R₅ = H ; R₆ = -(CH₂)₃-NC(O)OC(CH₃)₃-(CH₂)₄-NHC(O)OC(CH₃)₃; T₁ = T₂ = T₃ = T₄ = Hest appelé DX243BOC ou DXboc et est illustré dans le tableau 2.

Le substituant ou radical C(O)OC(CH₃)₃ est aussi appelé groupe fonctionnel tert-butoxycarbonyle ou Boc.

Le composé de formule (I) appartient au groupe des stéroïdes. La numérotation des atomes de carbone du composé de formule (I) suit donc la nomenclature définie par l'IUPAC dans Pure & Appl. Chem., Vol.61, No.10, pp.1783-1822,1989. La numérotation des atomes de carbone d'un composé appartenant au groupe des stéroïdes selon l'IUPAC est illustrée ci-dessous :

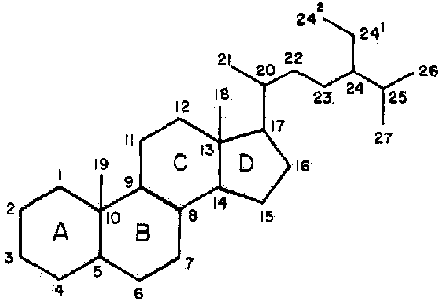

Les méthodes de préparation du composé de formule (I) ont déjà été décrites auparavant, et notamment dans DE MEDINA, P. et al. Synthesis of New ,the Treatment of Cancer and Neurodegenerative Diseases. Journal of Medicinal Chemistry, 52(23), 2009, pp. 7765-7777.

En outre, le composé peut posséder une ou plusieurs caractéristiques suivantes, considérées isolément ou en combinaison. Selon un mode de réalisation, le composé de formule (I) est défini par R₁ = OH, F, OCₙH₂ₙ₊₁, OC(O)R, OC(O)OR, OC(O)NHR ou OP(O)(OR)₂ avec R = H ou CₙH₂ₙ₊₁, avec 1≤ n ≤ 8 ;
R₂ = OH ;
R₃ = -NR₅R₆ ;
R₃ = H ;
R₆ = -(CH₂)₃NR₇(CH₂)₄NHR₇, -(CH₂)₃NHR₇, -(CH₂)₄NHR₇ , -(CH₂)₄N R₇(CH₂)₃ NHR₇, -(CH₂)₃N R₇(CH₂)₄N R₇(CH₂)₃NHR₇, -(CH₂)₂-imidazol-4-yl, -(CH₂)₂ - indol-3-yl, avec R₇ = H, C(O)OCH₃ ou C(O)OC(CH₃)₃;
Z₁ = 0 ou 1 ;
Z₂ = 0 ;
R₄ = H.

Selon un mode de réalisation, le composé de formule (I) est défini plus précisément par Z₁ = 0 , Z₂ = 0 ; R₁ = R₂ = OH ; R₄ = H; R₅ = H ; T₁ = T₂ = T₃ = T₄ = H, avec les autres radicaux R₃, R₆ et R₇tels que définis précédemment.

Selon un mode de réalisation, le composé de formule (I) est défini plus précisément par Z₁ = 0 ; Z₂ = 0 ; R₁ = R₂ = OH ; R₄ = H; R₅ = H; T₁ = T₂ = T₃ = T₄ = H, avec R₆ = -(CH₂)₄NH(CH₂)₃NHR₇ et R₇ = C(O)CH₃. Ce composé nommé DX249 est le 5α-hydroxy-6β-[3-(4-aminobutylacetamide)propylamino]cholestan-3β-ol .

Selon un mode de réalisation, le composé de formule (I) est défini plus précisément par Z₁ = 0 ; Z₂ = 0 ; R₁ = R₂ = OH ; R₄ = H ; R₅ = H ; T₁ = T₂ = T₃ = T₄ = H, avec R₆ = -(CH₂)₂-imidazol-4-yl. Ce composé nommé DX101 est le 5α-hydroxy-6β-[2-(1H-imidazol-4-yl)éthylamino]cholestan-3β-ol.

Selon un mode de réalisation, le composé de formule (I) est défini plus précisément par Z₁ = 0 ; Z₂ = 0 ; R₁ = R₂ = OH ; R₄ = H ; R₅ = H ; T₁ = T₂ = T₃ = T₄ = H, avec R₆ = -(CH₂)₃N R₇(CH₂)₄NHR₇, -(CH₂)₄NR₇(CH₂)₃NHR₇, -(CH₂)₃NR₇(CH₂)₄NH(CH₂)₃NHR₇ ; ou -(CH₂)₄NHR₇ ; et R₇ = H. Ces composés nommés respectivement DX243, DX245, DX301 et DX401 sont :
- 5α-hydroxy-6β-[3-(4-aminobutylamino)propylamino]cholestan-3β-ol (DX243)
- 5α-hydroxy-6β-[4-(3-aminobutylamino)propylamino]cholestan-3β-ol (DX245)
- 5α-hydroxy-6β-{3-[4-(3-aminopropylamino)butylamino]propylamino}cholest an-3β-ol (DX301)
- 5α-hydroxy-6β-(4-aminobutylamino)cholestan-3β-ol (DX401).

Selon un mode de réalisation, le composé de formule (I) est défini plus précisément par Z₁ = 0, Z₂ = 0 ; R₁ = R₂ = OH ; R₄ = H; R₅ = H; T₁ = T₂ = T₃ = T₄ = H, avec R₆ = -(CH₂)₃NR₇(CH₂)₄NHR₇ et R₇ = C(O)OC(CH₃)₃. Ce composé nommé DX243BOC est le 5α-hydroxy-6β-[3-(4-tert-butyloxycarbonylaminobutyl-tert - butyloxycarbonylamino)-propylamino]cholestan-3β-ol..

Selon un mode de réalisation, le composé de formule (I) est défini plus précisément par Z₁ = 1 ; Z₂ = 0 ; R₁ = R₂ = OH ; R₄ = H ; R₅ = H ; T₁ = T₂ = T₃ = T₄ = H, avec les autres radicaux R₃, R₆ et R₇tels que définis précédemment.

Selon un mode de réalisation, le composé de formule (I) est défini plus précisément par Z₁ = 1; Z₂ = 0 ; R₁ = R₂ = OH; R₄ = H; R₅ = H; T₁ = T₂ = T₃ = T₄ = H, avec R₆ = -(CH₂)₃NH(CH₂)₄NHR₇ ; -(CH₂)₄NH(CH₂)₃NHR₇ ; ou -(CH₂)₃NH(CH₂)₄NH(CH₂)₃ NHR₇ ; et R₇ = H. Ces composés nommés respectivement DX242, DX244 et DX302 sont les :
- 5α-hydroxy-6β-[3-(4-aminobutylamino)propylamino]cholest-7-en-3β-ol (DX242)
- 5α-hydroxy-6β-[4-(3-aminobutylamino)propylamino]cholest-7-en-3β-ol (DX244)
- 5α-hydroxy-6β-{3-[4-(3- aminopro-pylamino)butylamino]propylamino}cholest-7-en-3β-ol (DX302).

Selon un mode de réalisation, la pathologie liée à un déficit mitochondrial est due soit à un déficit de la dynamique et/ou du transport et/ou de l'activité mitochondriale. Le tableau 1 illustre la spécificité des déficits selon la pathologie.

**[Tableaux1]**

| | Prévalence (2020) | Essai clinique (2021) | | | |
|---|---|---|---|---|---|
| | | | Mitochondrie | | |
| | | | Dynamique (Fusion/ Fission) | Transport | Activité |
| Neuropathies | 300 millions *(2015)* | 2523 | ✔ | ✔ | ✔ |
| Lésions de la moelle épinière | 3,5 millions | 1377 | ✔ | ✔ | ✔ |
| Troubles du spectre autistique | 12 millions | 1172 | ✔ | ✔ | ✔ |
| Ataxie | 1 cas pour 10.000 *(2014)* | 299 | | ✔ | ✔ |
| Pathologies mentales liées à l'alcool (AUD) | 99,2 millions *(2016)* | 679 | ✔ | ✔ | ✔ |
| Sclérose en plaque | 3 millions | 2452 | | | ✔ |
| Migraine | 840 millions | 1143 | | | ✔ |
| Épilepsie | 50 millions | 1623 | | | |
| Troubles mitochondriaux liés à l'exposition de tabac | 1,3 milliards | 518 | ✔ | | ✔ |
| Syndrome MERRF | < 1 cas pour 100.000 | 1 | | | ✔ |
| Syndrome NARP | < 1 cas pour 10.000 | 2 | | | ✔ |

Pour le composé de formule (I) selon l'invention, la pathologie liée à un déficit mitochondrial est due à un déficit de la dynamique mitochondriale et/ou du transport mitochondrial et/ou de l'activité mitochondriale.

Dans un mode de réalisation, les pathologies liées à un déficit de la dynamique mitochondriale et du transport et de l'activité mitochondriale sont les neuropathies, les lésions de la moelle épinière, l'autisme, l'ataxie et les pathologies mentales liées à la consommation d'alcool.

Dans un autre mode de réalisation, les pathologies liées à un déficit de la dynamique mitochondriale et de l'activité mitochondriale sont la migraine, les troubles cérébraux et pulmonaires liés à la consommation du tabac et le syndrome de MERRF.

Dans un autre mode de réalisation, la pathologie liée à un déficit du transport et de l'activité mitochondriale est la sclérose en plaque.

Dans encore un autre mode de réalisation, la pathologie liée à un déficit de l'activité mitochondriale est le syndrome de NARP.

Un second objet selon l'invention est une composition pharmaceutique comprenant au moins un composé de formule (I) et/ou au moins un sel pharmaceutiquement acceptable d'au moins un composé de formule (I), pour son utilisation dans le traitement d'une pathologie liée à un déficit mitochondrial choisie parmi le groupe constitué par l'autisme, les lésions de la moelle épinière, la sclérose en plaque, l'épilepsie, la migraine, les pathologies mentales liées à la consommation d'alcool, l'ataxie, les neuropathies, les troubles cérébraux et pulmonaires liés à la consommation du tabac, le syndrome de MERRF et le syndrome de NARP.

La composition pour utilisation selon l'invention peut être administrée sous différentes formes adaptées à la pathologie à traiter. Ainsi, les différentes formes d'administration comprennent l'administration orale, topique, systémique, intraveineuse, sous-cutanée, intrapéritonéale, intramusculaire, transdermale ou trans-mucosale.

La composition selon l'invention peut se présenter sous toutes les formes galéniques normalement utilisées selon que la composition doit être ingérée, injectée ou appliquée sur la peau ou les muqueuses.

La composition selon l'invention pourra comprendre des ingrédients couramment utilisés dans ce type de formulations tels que des liants, des agents de saveurs, des conservateurs, des colorants, et pourront, dans le cas de compléments alimentaires ou de médicaments, se présenter sous la forme de comprimés, granulés ou gélules. Des formulations selon l'invention pourront également se présenter sous la forme de produits alimentaires tels que des boissons, ou encore sous la forme de suspensions ou de sirops.

Selon une première variante, les différentes préparations sont adaptées à l'administration topique et incluent les crèmes, les émulsions huile dans eau et eau dans huile, les laits, les pommades, les lotions, les huiles, les baumes, les solutions aqueuses ou hydro-alcooliques ou glycoliques, les sérums, les poudres, les patchs, les sprays ou tout autre produit pour application externe tel que par, exemple, les dispositifs médicaux ou les produits aérosol contenant également un agent propulseur sous pression.

Selon une deuxième variante, les différentes compositions sont adaptées à l'injection ; la composition peut se présenter sous forme d'une lotion aqueuse, huileuse ou sous forme de sérum.

Selon une troisième variante, les différentes compositions sont adaptées à l'ingestion ; la composition peut se présenter sous forme de capsules, de sirops, de granulés ou de comprimés.

Selon un mode de réalisation préférentiel, les compositions selon l'invention sont destinées plus particulièrement à une administration par voie topique. Ces compositions doivent donc contenir un milieu dermatologique acceptable, c'est-à-dire compatible avec la peau et les muqueuses, et couvrent toutes les formes dermatologiques. Ces compositions pourront notamment être sous forme de crèmes, émulsions huile-dans-eau, ou eau-dans-huile ou émulsions multiples, sérums, solutions, suspensions, gels, laits, lotions, sticks ou encore de poudres, et adaptées à une application sur la peau et les muqueuses. Ces compositions comprennent les excipients nécessaires à leur formulation, tels que solvants, émollients, épaississants, diluants, tensioactifs, anti-oxydants, agents bioactifs, colorants, conservateurs, parfums.

Les compositions selon l'invention comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs dermatologiques ou pharmaceutiques, des huiles essentielles, des vitamines, des acides gras essentiels, des tensioactifs, des polymères filmogènes, etc.

Dans tous les cas, l'homme de métier veillera à ce que ces adjuvants ainsi que leurs proportions soient choisis de telle manière à ne pas nuire aux propriétés avantageuses recherchées des compositions selon l'invention.

Selon un mode de réalisation, la composition pour utilisation selon l'invention est sous forme d'une solution aqueuse et présente une concentration de composé de formule (I) comprise entre 1 pmol.L⁻¹ et 1 mmol.L⁻¹, préférentiellement entre 10 pmol.L⁻¹ et 0,1 mmol.L⁻¹, plus préférentiellement entre 0,1 nmol.L⁻¹ et 1 µmol.L⁻¹.

### Brève description des figures

L'invention sera mieux comprise, et d'autres buts, détails, caractéristiques et avantages de celle-ci apparaîtront plus clairement au cours de la description suivante de plusieurs modes de réalisation particuliers de l'invention, donnés uniquement à titre illustratif et non limitatif, en référence aux dessins annexés.
[Fig.1] La [Fig.1] illustre les résultats d'un test utilisant des composés de formule (I) sur l'activité de neurones corticaux par mesure de la densité optique du sel de tétrazolium MTT (bromure de 3-(4,5-dimethylthiazol-2-yl)-2,5-diphenyl tetrazolium). NT signifie non traité. Les résultats permettent d'identifier l'activité métabolique relative des neurones corticaux avec ou sans composés de formule (I).
[Fig.2A] La [Fig.2A] illustre les résultats d'un test utilisant des composés de formule (I) sur l'activité de cellules Neuro-2a dans des conditions normales (non privées d'oxygène et de glucose), par mesure de la densité optique du sel de tétrazolium MTT. CTL signifie non traité. Les résultats permettent d'identifier l'activité métabolique relative des cellules Neuro-2a avec ou sans composés de formule (I).
[Fig.2B] La [Fig.2B] illustre et démontre l'effet bénéfique des composés selon la formule (I) sur l'activité métabolique de cellules Neuro-2a après une intoxication à l'éthanol (EtOH). [Fig.2C] La [Fig.2C] illustre et démontre l'effet bénéfique des composés selon la formule (I) sur l'activité des mitochondries dans des cellules Neuro-2a après une intoxication à l'éthanol (EtOH).
[Fig.3] La [Fig.3] illustre les résultats d'un test démontrant l'effet des composés de formule (I) sur la dynamique mitochondriale d'une lignée cellulaire.
[Fig.4] La [Fig.4] illustre les résultats d'un test démontrant l'inhibition de la fission mitochondriale avec les composés selon la formule (I). Une analyse qRT-PCR d'explants cochléaire traités avec le composé DX243 permet d'observer une diminution de l'ARNm de *drp1* après 24h de traitement au DX243 à 100 nM par rapport aux cochlées non-traités (NT).
[Fig.5] La [Fig.5] illustre les résultats d'un test d'évaluation de la dynamique mitochondriale et du stress mitochondrial dans des neurones dopaminergiques.
[Fig.6] La [Fig.6] illustre les résultats d'un test d'évaluation du stress mitochondrial dans une culture de neurones dopaminergiques.
[Fig.7] La [Fig.7] illustre les résultats d'un test d'évaluation du stress mitochondrial dans une culture de neurones hippocampiques.

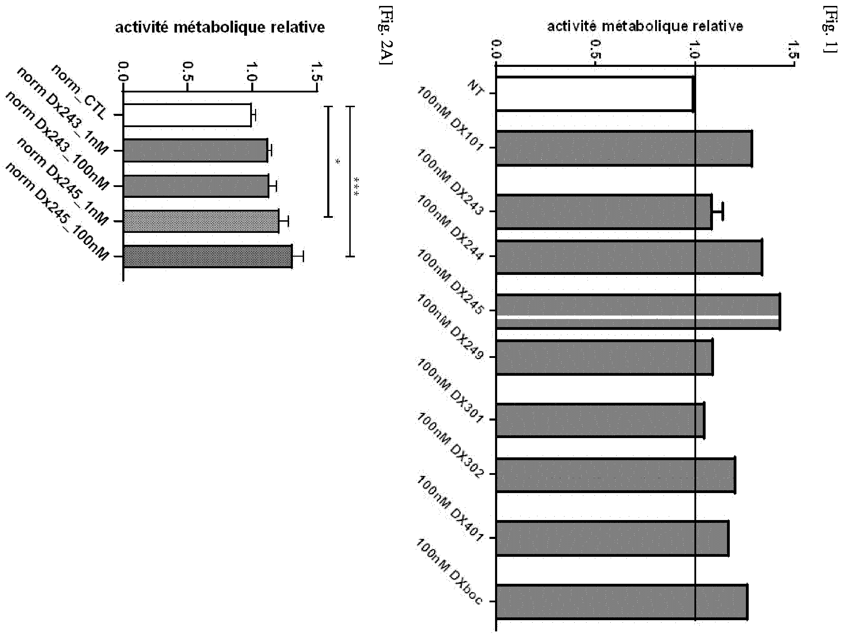

Sur les figures 2A, 2C, 3, 5 et 6, les étoiles indiquent la puissance statistique des résultats. Une étoile indique qu'il est certain à 95% que les résultats ne sont pas dus au hasard. La présence de 2 étoiles signifie qu'il est certains à 99% que les résultats ne sont pas dus au hasard, la présence de 3 étoiles indiquent qu'il est certain à 99,9% que les résultats ne sont pas dus au hasard et la présence de 4 étoiles indiquent qu'il est certain à 99,99% que les résultats ne sont pas dus au hasard.

### Description des modes de réalisation

Dans cette description, à moins qu'il n'en soit spécifié autrement, il est entendu que, lorsqu'un intervalle est donné, il inclut les bornes supérieures et inférieures dudit intervalle.

Dans la présente invention, on entend par :
« prévenir une pathologie» le fait d'éviter la survenue de maladies ou de traumatismes ou à maintenir et à améliorer la santé, on peut également parler de traitement préventif.
« améliorer une pathologie» le fait de réduire les symptômes de ladite pathologie, on peut également parler de traitement palliatif.
« traiter une pathologie» le fait de guérir une maladie, on peut également parler de traitement curatif.
« déficit mitochondrial » une baisse d'activité mitochondriale et/ou de la dynamique mitochondriale et/ou du transport axonal des mitochondries qui entraîne une perte de production d'énergie et l'accumulation de substances nocives pour l'organisme.
« activité mitochondriale » capacité qu'à la mitochondrie à réguler le métabolisme cellulaire et à produire de l'énergie (ATP) via sa chaine respiratoire.
« dynamique mitochondriale » capacité qu'ont les mitochondries à fusionner ou fissioner afin de préserver leur morphologie et leur taille et/ou pour permettre l'augmentation de la quantité (masse) mitochondriale dans les cellules.
« Transport axonal des mitochondries» capacité qu'ont les mitochondries à se déplacer afin d'assurer la demande en énergie nécessaire à un endroit précis de la cellule.
« fission mitochondriale » division d'une mitochondrie en 2 mitochondries distinctes.

Afin de démontrer que les composés de formule (I) ont une influence sur la fonction mitochondriale des cellules, il a été réalisé différentes expériences prouvant l'effet bénéfique desdits composés sur la fonction mitochondriale en générale, à travers leurs effets sur le nombre, la dynamique, le stress, le transport ou l'activité des mitochondries. Il est décrit ci-après plusieurs protocoles expérimentaux qui mettent en évidence l'effet bénéfique des composés de formule (I), notamment ceux indiqués dans le tableau 2 ci-après, dans différentes conditions et différents modèles expérimentaux.

**Tableau 2 : Liste des composés de formules I utilisés dans les exemples expérimentaux**

| Nom | Structure | Nom | Structure |
|---|---|---|---|
| **DX101** | 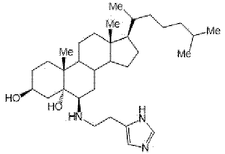 | **DX249** | 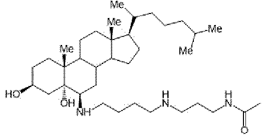 |
| **DX243** | 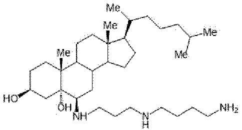 | **DX301** | 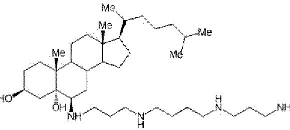 |
| **DX242** | 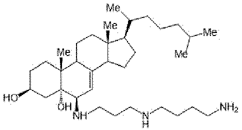 | **DX302** | 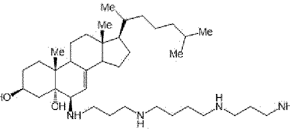 |
| **DX245** | 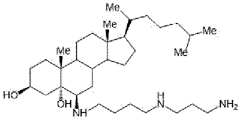 | **DX401** | 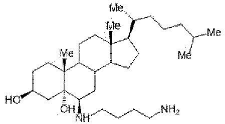 |
| **DX244** | 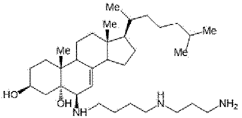 | **DX243BOC** | 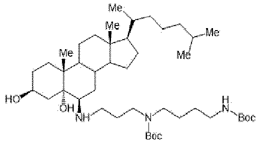 |

Les concentrations ou molarités des composés sont exprimées en mole par litre dont le symbole est mol.L⁻¹ ou M.

### Exemple 1 : Test de l'activité métabolique relative sur des neurones corticaux

Il a été mis au point un protocole permettant d'obtenir une culture primaire de neurones corticaux à partir de cellules prélevées sur des cerveaux d'embryons de souris de type sauvage. L'étape 1 a consisté à prélever les cortex embryonnaires pour mettre les neurones corticaux dans des conditions de culture adéquates. La seconde étape a consisté à mettre en culture les cellules prélevées dans un milieu Neurobasal^{™} Medium (Ref. 21103049 ThermoFisher Scientific) auquel de la L-Glutamine et du B27 supplement 50X (Ref. 17504044 ThermoFisher Scientific) ont été ajoutés. Les neurones issus de la culture primaire sont ensuite isolés et purifiés. Les conditions de culture précitées permettent d'obtenir une culture purifiée en neurones à partir de la dissociation des cortex embryonnaires.

Un test basé sur l'activité métabolique des neurones a été réalisé pour évaluer l'effet des composés de formule (I) sur cette activité métabolique. Ce test est basé sur l'utilisation du sel de tétrazolium MTT (bromure de 3-(4,5-dimethylthiazol-2-yl)-2,5-diphenyl tetrazolium). Le tétrazolium est réduit par la succinate déshydrogénase mitochondriale des cellules vivantes actives, en formazan, précipité de couleur violette. La quantité de précipité formée est proportionnelle à l'activité métabolique présente au sein de la culture. Ainsi, un dosage de la densité optique à 550 nm par spectroscopie permet de connaître la quantité relative d'activité métabolique et par conséquent l'activité mitochondriale. Les résultats obtenus ont été rapportés dans la [Fig.1]. Ce test sur des cultures de neurones corticaux a été effectué avec différents composés en référence avec le Tableau 2 (DX101, DX243, DX244, DX245, DX249, DX301, DX302 et DX401) utilisés à une même concentration (100 nM).

En référence à la [Fig.1], ce test permet de mettre en évidence un effet positif des composés testés de formule (I) sur l'activité mitochondriale par rapport à des neurones non-traités.

### Exemple 2 : Test de l'activité métabolique relative sur des neurones Neuro-2a

Le même test décrit à l'exemple 1 a été effectué sur des cellules de la lignée Neuro-2a - lignée cellulaire de neuroblastome de souris. En condition physiologique une fois mise en culture et à une confluence d'environ 50%, les cellules Neuro-2a ont été mises en présence de 2 concentrations des composés DX243 et DX245 pendant 24 h. Un test MTT réalisé à la suite de ces 24 h de traitement a permis de mettre en évidence que le composé DX243 mais surtout le composé DX245 permettait d'augmenter l'activité métabolique des cellules Neuro-2a, comme représenté à la [Fig.2A].

### Exemple 3 : Test de l'activité métabolique après une intoxication à l'éthanol (EtOH)

Le même test décrit à l'exemple 1 a été effectué sur des cellules de la lignée Neuro-2a - lignée cellulaire de neuroblastome de souris. Il est démontré qu'une intoxication pendant 24 h à l'éthanol (EtOH) à forte concentration (500 mM) au sein de cette lignée cellulaire diminuait l'activité métabolique de ces cellules (χ = P value < 0.0001 par rapport au contrôle). Cette activité métabolique est partiellement récupérée avec un traitement de 24 h avec 1 nM de composé DX243.

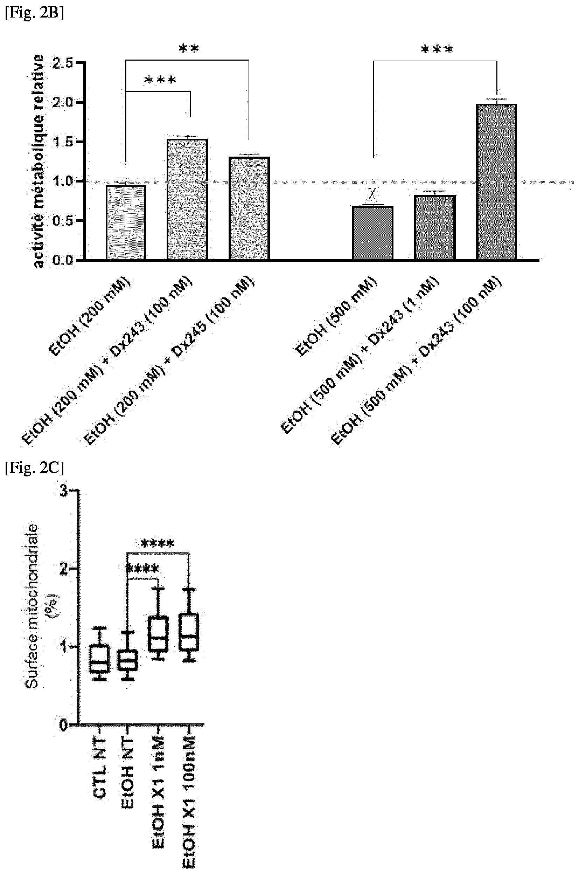

De façon intéressante, un traitement de 24 h avec 100 nM de composé DX243 ou de composé DX245 donne lieu à une activité métabolique significativement plus importante par rapport aux cellules non-traitées, après une intoxication à l'EtOH (200 mM et 500 mM), comme illustré à la [Fig.2B].

Des effets du composé DX243 sur les mitochondries dans des cellules Neuro-2a après une intoxication à l'éthanol sont présentés à la [Fig.2C]. Cette figure met en avant l'augmentation de la surface mitochondriale en présence du composé DX243, étudiée à l'aide d'un MitoTracker, après une intoxication de 24 h par de l'éthanol (500 mm).

### - Exemple 4 : Test de la dynamique mitochondriale sur les lignées cellulaires SHSY5Y et C2C12

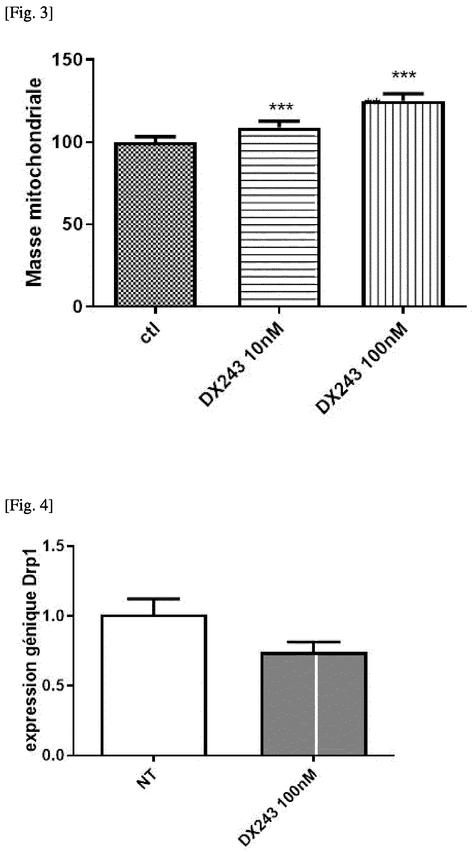

Une lignée de cellules de neuroblastome humain (SHSY5y) ainsi qu'une lignée de cellules du myoblaste (C2C12) ont été mises en culture dans un milieu DMEM^{™} (Ref. 21068028 ThermoFisher Scientific). Une fois en culture, ces 2 lignées cellulaires ont été traitées pendant 24h avec différentes concentrations du composé DX243. Une série d'images a été réalisée avec un marquage Mitotracker^{™} (Ref, M7512,ThermoFisher Scientific), qui permet de mettre en évidence et visualiser les mitochondries présentes au sein de ces cellules et de pouvoir en quantifier la quantité. Cette quantification de la masse mitochondriale révélée à l'aide du marquage Mitotracker a mis en évidence un effet du composé DX243 sur la dynamique mitochondriale (augmentation de la quantité (masse) mitochondriale, comme illustré à la [Fig.3].

### Exemple 5 : Test d'évaluation d'inhibition de la fission mitochondriale sur des explants cochléaires

Il a été mis au point un modèle d'explant cochléaires. Pour obtenir des cochlées, organe de l'audition, en culture, des cochlées de souriceaux de 3 jours ont été micro-disséquées. Ces cochlées sont ensuite placées dans du milieu DMEM avec ajout de Glucose (0,5%), N1 (0,5%), Insuline (0,25%) et Peniciline (0,1%) , pendant 24h. Ensuite 100nM du composé DX243 est ajouté à ce milieu de culture pendant 24h.

Les cochlées sont ensuite récupérées et l'ARN en est extrait afin de pouvoir analyser la modulation de plusieurs gènes suite au traitement avec le composé DX243. Il est ainsi observé que le traitement d'explants cochléaires avec le composé DX243 (100 nM) entraine une inhibition de l'expression du gène de fission mitochondriale *drp1,* comme illustré à la [Fig.4] . Ce test démontre le rôle bénéfique du composé DX243 sur les mitochondries, notamment en inhibant la fission mitochondriale.

### Exemple 6 : Test d'évaluation de la dynamique mitochondriale et du stress mitochondrial dans neurones dopaminergiques

Les effets mitochondriaux du composé DX243 ont été testés dans un modèle de neurones dopaminergiques traités au MPP+ (1-méthyl-4-phénylpyridinium), une toxine mitochondriale qui augmente le stress mitochondrial et diminue le nombre de mitochondries. Le MPP+ est connu pour ses effets délétères sur la mitochondrie (augmentation du stress mitochondrial mesuré par la quantité de cytochrome C, diminution du nombre de mitochondrie mesuré à l'aide du test Mitotracker). Les neurones dopaminergiques ont été obtenus à partir de la microdissection du mésencéphale d'embryons de rats cultivé avec du milieu Neurobasal^{™} Medium (Ref. 21103049 ThermoFisher Scientific) supplémenté avec du B27 supplement 50X (Ref. 17504044 ThermoFisher Scientific), de la peniciline/streptomycin, de la L-Glutamine ainsi que du BDNF (Brain-derived neurotrophic factor) et du GDNF (Glial cells-derived neurotrophic factor). Ces neurones sont mis en culture pendant 5 jours puis traités pendant 48 h avec du MPP+. Ces neurones sont également en présence de différentes concentrations du composé DX243 pendant les 48 h de traitement au MPP+ ainsi que 48 h après le retrait du MPP+.

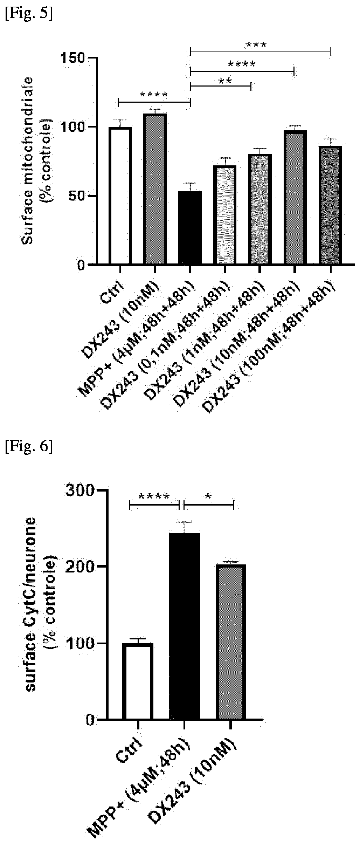

Les résultats démontrent que le composé DX243 est capable d'augmenter et de restaurer la masse mitochondriale dans ces neurones dopaminergiques ayant perdu une partie de cette masse suite à un traitement au MPP+, comme illustré dans la [Fig.5] .

De plus, 10 nM du composé DX243 entraine également une diminution du stress mitochondrial engendré par le traitement au MPP+, comme illustré à la [Fig.6].

### Exemple 7 : Test d'évaluation de la dynamique mitochondriale et du stress mitochondrial dans neurones hippocampiques

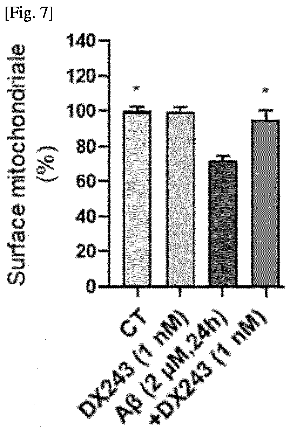

Les effets mitochondriaux du composé DX243 ont été testés dans un modèle de neurones hippocampiques traités au peptide Aβ 1-42, lequel peptide induit un stress mitochondrial (représenté par une diminution de la surface mitochondriale). Il est démontré que le composé DX243 (1 nM) est capable de restaurer cette diminution de surface mitochondriale, comme illustré à la [Fig.7].

## Revendications

1. Composé de formule (I) : 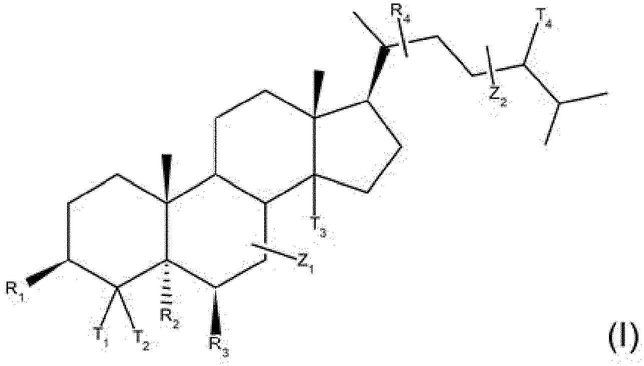 dans laquelle
R₁ = OH, F, OCₙH₂ₙ₊₁, OC(O)R, OC(O)OR, OC(O)NHR ou OP(O)(OR) ₂ avec R = H ou CₙH₂ₙ₊₁, avec 1≤ n ≤ 8 ;
R₂ = H ou OH ;
R₃ = -NR₅R₆ ;
R₅ étant H ou -(CH₂)₃NH₂ ; et
R₆ étant pris dans le groupe formé par -(CH₂)₃NR₇(CH₂)₄NHR₇ , -(CH₂) ₃NHR₇ , -(CH₂)₄NHR₇ , -(CH₂)₄N R₇(CH₂)₃NHR₇ , -(CH₂)₃N R₇(CH₂)₄N R₇(CH₂)₃NHR₇ , -(CH₂)₂-imidazol-4-yl, -(CH₂)₂-indol-3-yl, avec R₇ = H, C(O)OCH₃ ou C(O)OC(CH₃)₃; et
R₄ = H ou OH en position 20, 22, 24, 25, 26 ou 27, positionné de façon à créer un centre asymétrique de configuration R ou S ;
Z₁ et Z₂ représentent chacun le nombre de doubles liaisons entre les atomes de carbone C7 et C8 et C22 et C23 respectivement (soit 0 soit 1) ; T₁, T₂ et T₃ = H ou CH₃ indépendamment les uns des autres ; T₄ = H, CH₃, C₂H₅ positionné de façon à obtenir un centre asymétrique de configuration R ou S en position 24 ;
pour son utilisation dans le traitement d'une pathologie liée à un déficit mitochondrial choisie parmi le groupe constitué par l'autisme, les lésions de la moelle épinière, la sclérose en plaque, l'épilepsie, la migraine, les pathologies mentales liées à la consommation d'alcool, l'ataxie, les neuropathies, les troubles cérébraux et pulmonaires liés à la consommation du tabac, le syndrome de MERRF et le syndrome de NARP.

2. Composé pour utilisation selon la revendication 1, dans lequel le composé de formule (I) est défini par :
R₁ = OH, F, OCₙH₂ₙ₊₁, OC(O)R, OC(O)OR, OC(O)NHR ou OP(O)(OR) ₂ avec R = H ou CₙH₂ₙ₊₁, avec 1≤ n ≤ 8 ;
R₂ = OH ; R₃ = -NR₅R₆ ;
R₅ = H ;
R₆ = -(CH₂)₃NR₇(CH₂)₄NHR₇ , -(CH₂)₃NHR₇ ,-(CH₂)_{4N}HR₇ , -(CH₂)₄N R₇(CH₂)₃NHR₇ , -(CH₂)₃NR₇(CH₂)₄N R₇(CH₂)₃NHR₇ , -(CH₂)₂ - imidazol-4-yl, -(CH₂)₂-indol-3-yl, avec R₇ = H, C(O)OCH₃ ou C(O)OC(CH₃)₃; Z₁ = 0 ou 1 ;
Z₂ = 0 ; R₄ = H .

3. Composé pour utilisation selon la revendication 1 ou 2, dans lequel le composé de formule (I) est défini par Z₁ = 0, Z₂ = 0 ; R₁ = R₂ = OH ; R₄ = H ; R₃ = H ; T₁ = T₂ = T₃ = T₄ = H.

4. Composé pour utilisation selon la revendication 3, dans laquelle le composé de formule (I) est défini par R₆ = -(CH₂)₄NH(CH₂)₃NHR₇ avec R₇ = C(O)CH₃ et est le composé 5α-hydroxy-6β-[3-(4-aminobutylacetamide)propylamino]cholestan-3β-ol.

5. Composé pour utilisation selon la revendication 3, dans lequel le composé de formule (I) est défini par R₆ = -(CH₂)₂NHR₇ avec R₇ = imidazol-4-yl et est le 5α-hydroxy-6β-[2-(1H-imidazol-4-yl)éthylamino]cholestan-3β-ol.

6. Composé pour utilisation selon la revendication 3, dans lequel le composé de formule (I) est défini par R₆ = -(CH₂)₃NR₇(CH₂)₄NHR₇, -(CH₂)₄NR₇ (CH₂)₃NHR₇, -(CH₂)₃NR₇(CH₂)₄NR₇(CH₂)₃NHR₇ ; ou -(CH ₂)₄NHR₇ ; et R₇ = H et est respectivement ( 5α-hydroxy-6β-[3-(4-aminobutylamino)propylamino]cholestan-3β-ol, 5α-hydroxy-6β-[4-(3-aminobutylamino)propylamino]cholestan-3β-ol, 5α-hydroxy-6β-{3-[4-(3-aminopropylamino)butylamino]propylamino}c holestan-3β-ol, 5α-hydroxy-6β-(4-aminobutylamino)cholestan-3β-ol.

7. Composé pour utilisation selon la revendication 3, dans lequel le composé de formule (I) est défini par R₆ = -(CH₂)₃NR₇(CH₂)₄NHR₇ et R ₇ = C(O)OC(CH₃)₃ et est 5α-hydroxy-6β-[3-(4-*tert* - butyloxycarbonylaminobutyl-tert-butyloxycarbonylamino)propylamino] cholestan-3β-ol.

8. Composé pour utilisation selon la revendication 2, dans lequel le composé de formule (I) est défini par Z₁ = 1 et Z₂ = 0 ; R₁ = R₂ = OH ; R ₄ = H ; R₅ = H ; T₁ = T₂ = T₃ = T₄ = H.

9. Composé pour utilisation selon la revendication 8, dans lequel le composé de formule (I) est défini par R₆ = -(CH₂)₃NR₇(CH₂)₄NHR₇ ; -(CH₂)₄NR₇(CH₂)₃NHR₇ ou -(CH₂)₃NR₇(CH₂)₄NR₇(CH₂)₃NHR₇ ; et R₇ = H et est respectivement ( 5α-hydroxy-6β-[3-(4-aminobutylamino)propylamino]cholest-7-en-3β-ol 5α-hydroxy-6β-[4-(3-aminobutylamino)propylamino]cholest-7-en-3β-ol 5α-hydroxy-6β-{3-[4-(3-aminopropylamino)butylamino]propylamino }c holest-7-en-3β-ol.

10. Composé pour utilisation selon l'une des revendications 1 à 9, dans laquelle la pathologie liée à un déficit mitochondrial est due à un déficit de la dynamique mitochondriale et/ou du transport mitochondrial et/ou de l'activité mitochondriale.

11. Composition pharmaceutique comprenant au moins un composé de formule (I) et/ou au moins un sel pharmaceutiquement acceptable d'au moins un composé de formule (I) selon l'une des revendications 1 à 9, pour son utilisation dans le traitement d'une pathologie liée à un déficit mitochondrial choisie parmi le groupe constitué par l'autisme, les lésions de la moelle épinière, la sclérose en plaque, l'épilepsie, la migraine, les pathologies mentales liées à la consommation d'alcool, l'ataxie, les neuropathies, les troubles cérébraux et pulmonaires liés à la consommation du tabac, le syndrome de MERRF et le syndrome de NARP.

12. Composition pour utilisation selon la revendication 11, **caractérisée en ce qu'**elle est sous forme d'une solution aqueuse et présente une concentration de composé de formule (I) comprise entre 1 pmol.L⁻¹ et 1 mmol.L⁻¹, préférentiellement entre 10 pmol.L⁻¹ et 0,1 mmol.L⁻¹, plus préférentiellement entre 0,1 nmol.L⁻¹ et 1 µmol.L⁻¹.

## Patentansprüche

1. Verbindung der Formel: 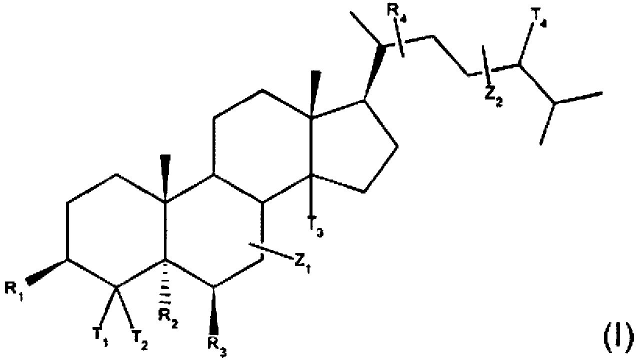 wobei
R₁ = OH, F, OCₙH₂ₙ₊₁, OC(O)R, OC(O)OR, OC(O)NHR oder OP(O)(OR)₂ mit R
= H oder CₙH₂ₙ₊₁, mit 1≤ n ≤ 8;
R₂ = H oder OH;
R₃ = -NR₅R₆;
R₅ ist H oder -(CH₂)₃NH₂; und
R₆ ausgewählt ist aus der Gruppe bestehend aus -(CH₂)₃NR₇(CH₂)₄NHR₇, - (CH₂)₃NHR₇,-(CH₂)₄NHR₇,-(CH₂)₄N R₇(CH₂)₃NHR₇, -(CH₂)₃,N R₇(CH₂)₄NR₇(CH₂)₃NHR₇, -(CH₂)₂-Imidazol-4-yl, -(CH₂)₂-Indol-3-yl, mit R₇ = H, C(O)OCH₃ oder C(O)OC(CH₃)₃; und
R₄ = H oder OH in Position 20, 22, 24, 25, 26 oder 27, so angeordnet , dass ein asymmetrisches Zentrum mit R oder S Konfiguration gebildet ist;
Z₁ und Z₂ jeweils die Anzahl der Doppelbindungen zwischen den Kohlenstoffatomen C7 und C8 beziehungsweise C22 und C23 (entweder 0 oder 1) darstellen; T₁, T₂, T₃ = H oder CH₃ unabhängig voneinander; T₄ = H, CH₃, C₂H₅, so angeordnet, dass ein asymetrisches Zentrum mit R oder S Konfigurationen in Position 24 entsteht;
zur Verwendung bei der Behandlung einer Erkrankung im Zusammenhang mit einem mitochondrialen Defizit, ausgewählt aus der Gruppe bestehend aus Autismus, Rückenmarksverletzungen, Multipler Sklerose, Epilepsie, Migräne, psychischen Erkrankungen im Zusammenhang mit Alkoholkonsum, Ataxie, Neuropathien, durch Tabakkonsum bedingte Hirn- und Lungenerkrankungen, MERRF-Syndrom und NARP-Syndrom.

2. Verbindung zur Verwendung gemäß Anspruch 1, wobei die Verbindung der der Formel (I) definiert ist durch:
R₁ = OH, F, OCₙH₂ₙ₊₁, OC(O)R, OC(O)OR, OC(O)NHR oder OP(O)(OR)₂ mit R = H oder CₙH₂ₙ₊₁, mit 1≤ n ≤ 8;
R₂ = OH; R₃ = -NR₅R₆;
R₅ = H;
R₆ = -(CH₂)₃NR₇(CH₂)₄NHR₇, -(CH₂)₃NHR₇, -(CH₂)₄NHR₇, - (CH₂)₄NR₇(CH₂)₃NHR₇, -(CH₂)₃NR₇(CH₂)₄N R₇(CH₂)₃NHR₇, -(CH₂)₂ -Imidazol-4-yl, -(CH₂)₂-Indol-3-yl, mit R₇ = H, C(O)OCH₃ oder C(O)OC(CH₃)₃; Z₁ = 0 oder 1; Z₂ = 0; R₄ = H.

3. Verbindung zur Verwendung gemäß Anspruch 1 oder 2, wobei die Verbindung der der Formel (I) definiert ist durch Z₁ = 0, Z₂ = 0; R₁ = R₂ = OH; R₄ = H; R₅ = H; T₁ = T₂= T₃ = T₄ = H.

4. Verbindung zur Verwendung gemäß Anspruch 3, wobei die Verbindung der der Formel (I) definiert ist durch R₆ = -(CH₂)₄NH(CH₂)₃NHR₇ mit R₇ = C(O)CH₃ und ist die Verbindung
5α-Hydroxy-6β-[3-(4- Aminobutylacetamid)propylamino]cholestan-3β-ol.

5. Verbindung zur Verwendung gemäß Anspruch 3, wobei die Verbindung der der Formel (I) definiert ist durch R₆ = -(CH₂)₂NHR₇ mit R₇ = Imidazol-4-yl und ist der
5α-Hydroxy-6β-[2-(1H-Imidazol-4-yl)Ethylamino]cholestan-3β-ol.

6. Verbindung zur Verwendung gemäß Anspruch 3, wobei die Verbindung der der Formel (I) definiert ist durch R₆ = -(CH₂)₃NR₇(CH₂)₄NHR₇, - (CH₂)₄NR₇(CH₂)₃NHR₇,-(CH₂)₃NR₇(CH₂)₄NR₇(CH₂)₃NHR₇; oder-(CH₂)₄NHR₇; und R₇ = H und ist jeweils ( 5α-Hydroxy-6β-[3-(4-Aminobutylamino)propylamino]cholestan-3β-ol, 5α-Hydroxy-6β-[4-(3-Aminobutylamino)propylamino]cholestan-3β-ol, 5α-Hydroxy-6β-{3-[4-(3-Aminopropylamino)butylamino]propylamino} cholestan-3β-ol, 5α-Hydroxy-6β-(4-Aminobutylamino)cholestan-3β-ol.

7. Verbindung zur Verwendung gemäß Anspruch 3, wobei die Verbindung der der Formel (I) definiert ist durch R₆ = -(CH₂)₃NR₇(CH₂)₄NHR₇ und R₇ = C(O)OC(CH₃)₃ und ist 5α-Hydroxy-6β-[3-(4-*tert* - Butyloxycarbonylami-nobutyl-*tert*-butyloxycarbonylamino)propylamino]cholestan-3β-ol.

8. Verbindung zur Verwendung gemäß Anspruch 2, wobei die Verbindung der der Formel (I) definiert ist durch Z₁ = 1 und Z₂ = 0; R₁ = R₂ = OH; R₄ = H; R₅ = H; T₁ = T₂ = T₃ = T₄ = H.

9. Verbindung zur Verwendung gemäß Anspruch 8, wobei die Verbindung der der Formel (I) definiert ist durch R₆ = -(CH₂)₃NR₇(CH₂)₄NHR₇: - (CH₂)₄NR₇(CH₂)₃NHR₇ oder -(CH₂)₃NR₇(CH₂)₄NR₇(CH₂)₃NHR₇; und R₇ = H und ist jeweils( 5α-Hydroxy-6β-[3-(4-Aminobutylamino)propylamino]cholest-7-en-3β-ol 5α-Hydroxy-6β-[4-(3-Aminobutylamino)propylamino]cholest-7-en-3β-ol 5α-Hydroxy-6β-{3-[4-(3- Aminopropylamino)butylamino]propylamino} cho-lest-7-en-3β-ol.

10. Verbindung zur Verwendung gemäß einem der Ansprüche 1 bis 9, wobei die mit einem mitochondrialen Defizit verbundene Erkrankung auf einem Defizit der mitochondrialen Dynamik und/oder des mitochondrialen Transports und/oder der mitochondrialen Aktivität beruht.

11. Pharmazeutische Zusammensetzung, die mindestens eine Verbindung der Formel (I) und/oder mindestens ein pharmazeutisch akzeptables Salz mindestens einer Verbindung der Formel (I) gemäß einem der Ansprüche 1 bis 9 umfasst, zur Verwendung bei der Behandlung einer Erkrankung, die mit einem mitochondrialen Defizit verbunden ist, ausgewählt aus der Gruppe bestehend aus Autismus, Rückenmarksverletzungen, Multiple Sklerose, Epilepsie, Migräne, alkoholbedingte psychische Erkrankungen, Ataxie, Neuropathien, tabakbedingte Gehirn- und Lungenerkrankungen, MERRF-Syndrom und NARP-Syndrom ausgewählt ist.

12. Zusammensetzung zur Verwendung gemäß Anspruch 11, **dadurch gekennzeichnet, dass** sie eine wässrige Lösung ist und eine Konzentration der Verbindung der Formel (I) zwischen 1 pmol.L⁻¹ und 1 mmol.L⁻¹, vorzugsweise zwischen 10 pmol.L ⁻¹ und 0,1 mmol.L⁻¹, noch bevorzugter zwischen 0,1 nmol.L⁻¹ und 1 µmol.L⁻¹.

## Claims

1. A compound of formula (I): 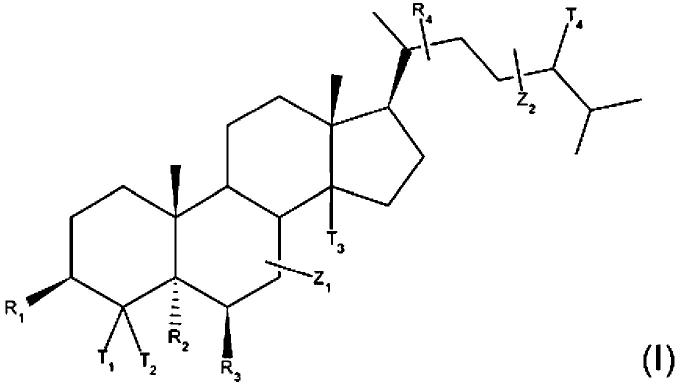 in which
R₁ = OH, F, OCₙH₂ₙ₊₁, OC(O)R, OC(O)OR, OC(O)NHR or OP(O)(OR)₂ with R = H or CₙH₂ₙ₊₁, with 1 ≤ n ≤ 8;
R₂ = H or OH;
R₃ = -NR₅R₆;
R₅ being H or -(CH₂)₃NH₂; and
R₆ being included in the group formed by -(CH₂)₃NR₇(CH₂)₄NHR₇, -(CH₂)₃NHR₇, - (CH₂)₄NHR₇, -(CH₂)₄N R₇(CH₂)₃NHR₇, -(CH₂)₃N R₇(CH₂)₄N R₇(CH₂)₃NHR₇, -(CH₂)₂-imidazol-4-yl, -(CH₂)₂-indol-3-yl, with R₇ = H, C(O)OCH₃ or C(O)OC(CH₃)₃; and R₄ = H or OH in position 20, 22, 24, 25, 26 or 27, positioned to create an asymmetric center of R or S configuration;
Z₁ and Z₂ each represent the number of double bonds between carbon atoms C7 and C8 and C22 and C23 respectively (either 0 or 1); T₁, T₂ and T₃ = H or CH₃ independently of each other; T₄ = H, CH₃, C₂H₅ positioned to obtain an asymmetric center of R or S configuration in position 24;
for its use in the treatment of a mitochondrial deficiency-related pathology chosen from the group consisting of autism, spinal cord injury, multiple sclerosis, epilepsy, migraine, alcohol-related mental pathologies, ataxia, neuropathies, smoking-related cerebral and pulmonary disorders, MERRF syndrome and NARP syndrome.

2. The compound for its use as claimed in claim 1, in which the compound of formula (I) is defined by:
R₁ = OH, F, OCₙH₂ₙ₊₁, OC(O)R, OC(O)OR, OC(O)NHR or OP(O)(OR)₂ with R = H or CₙH₂ₙ₊₁, with 1 ≤ n ≤ 8;
R₂ = OH;
R₃ = -NR₅R₆;
R₅ = H;
R₆ = -(CH₂)₃NR₇(CH₂)₄NHR₇, -(CH₂)₃NHR₇, -(CH₂)₄NHR₇, -(CH₂)₄N R₇(CH₂)₃NHR₇, -(CH₂)₃N R₇(CH₂)₄N R₇(CH₂)₃NHR₇, -(CH₂)₂-imidazol-4-yl, -(CH₂)₂-indol-3-yl, with R₇ = H, C(O)OCH₃ or C(O)OC(CH₃)₃;
Z₁ = 0 or 1 ;
Z₂ = 0;
R₄ = H.

3. The compound for the use as claimed in claim 1 or 2, in which the compound of formula (I) is defined by Z₁ = 0, Z₂ = 0; R₁ = R₂ = OH; R₄ = H; R₅ = H; T₁ = T₂ = T₃ = T₄ = H.

4. The compound for the use as claimed in claim 3, in which the compound of formula (I) is defined by R₆ = -(CH₂)₄NH(CH₂)₃NHR₇ with R₇ = C(O)CH₃ and is the compound 5α-hydroxy-6β-[3-(4-aminobutylacetamide)propylamino]cholestan-3β-ol.

5. The compound for the use as claimed in claim 3, in which the compound of formula (I) is defined by R₆ = -(CH₂)₂NHR₇ with R₇ = imidazol-4-yl and is 5α-hydroxy-6β-[2-(1H-imidazol-4-yl)ethylamino]cholestan-3β-ol.

6. The compound for the use as claimed in claim 3, in which the compound of formula (I) is defined by R₆ = -(CH₂)₃NR₇(CH₂)₄NHR₇, -(CH₂)₄NR₇ (CH₂)₃NHR₇, - (CH₂)₃NR₇(CH₂)₄NR₇(CH₂)₃NHR₇; or -(CH₂)₄NHR₇; and R₇ = H and is, respectively, 5α-hydroxy-6β-[3-(4-aminobutylamino)propylamino]cholestan-3β-ol, 5α-hydroxy-6β-[4-(3-aminobutylamino)propylamino]cholestan-3β-ol, 5α-hydroxy-6β-{3-[4-(3-aminopropylamino)butylamino]propylamino}cholestan-3β-ol, 5α-hydroxy-6β-(4-aminobutylamino)cholestan-3β-ol.

7. The compound for the use as claimed in claim 3, in which the compound of formula (I) is defined by R₆ = -(CH₂)₃NR₇(CH₂)₄NHR₇ and R₇ = C(O)OC(CH₃)₃ and is 5α-hydroxy-6β-[3-(4-*tert*-butyloxycarbonylaminobutyl-*tert-*butyloxycarbonylamino)propylamino]cholestan-3β-ol.

8. The compound for the use as claimed in claim 2, in which the compound of formula (I) is defined by Z₁ = 1 and Z₂ = 0; R₁ = R₂ = OH; R₄ = H; R₅ = H; T₁ = T₂ = T₃ = T₄ = H.

9. The compound for the use as claimed in claim 8, in which the compound of formula (I) is defined by R₆ = -(CH₂)₃NR₇(CH₂)₄NHR₇; -(CH₂)₄NR₇(CH₂)₃NHR₇ or - (CH₂)₃NR₇(CH₂)₄NR₇(CH₂)₃NHR₇; and R₇ = H and is, respectively, 5α-hydroxy-6β-[3-(4-aminobutylamino)propylamino]cholest-7-en-3β-ol, 5α-hydroxy-6β-[4-(3-aminobutylamino)propylamino]cholest-7-en-3β-ol, 5α-hydroxy-6β-{3-[4-(3-aminopropylamino)butylamino]propylamino}cholest-7-en-3β-ol.

10. The compound for the use as claimed in one of claims 1 to 9, in which the mitochondrial deficiency-related pathology is due to a deficiency in mitochondrial dynamics and/or mitochondrial transport and/or mitochondrial activity.

11. A pharmaceutical composition comprising at least one compound of formula (I) and/or at least one pharmaceutically acceptable salt of at least one compound of formula (I) as claimed in one of claims 1 to 9, for its use in treating a mitochondrial deficiency-related pathology chosen from the group consisting of autism, spinal cord injury, multiple sclerosis, epilepsy, migraine, alcohol-related mental pathologies, ataxia, neuropathies, smoking-related cerebral and pulmonary disorders, MERRF syndrome and NARP syndrome.

12. The composition for use as claimed in claim 11, **characterized in that** it is in the form of an aqueous solution and has a concentration of compound of formula (I) of between 1 pmol.L⁻¹ and 1 mmol.L⁻¹, preferentially between 10 pmol.L⁻¹ and 0.1 mmol.L⁻¹, more preferentially between 0.1 nmol.L⁻¹ and 1 µmol.L⁻¹.
